# EUROPÄISCHE PATENTSCHRIFT

(11) **EP 1 858 405 B1**
(45) Veröffentlichungstag und Bekanntmachung des Hinweises auf die Patenterteilung: **14.08.2013**
(21) Anmeldenummer: 06700765.8
(22) Anmeldetag: 21.01.2006
(51) Int. Cl.: A61B 5/03

(54) **VORRICHTUNG ZUR MESSUNG VON HIRNPARAMETERN**
DEVICE FOR MEASURING BRAIN PARAMETERS
DISPOSITIF DE MESURE DE PARAMETRES CEREBRAUX

(30) Priorität: 24.02.2005 DE 102005008454
(43) Veröffentlichungstag der Anmeldung: 28.11.2007
(73) Patentinhaber: RAUMEDIC AG, 95233 Helmbrechts (DE)
(72) Erfinder: REICHENBERGER, Robert, 95632 Wunsiedel (DE); KUNZE, Gerd, 08297 Zwönitz (DE); GÖHLER, Karl-Heinz, 08279 Zwönitz (DE)
(74) Vertreter: Hofmann, Matthias
(86) Internationale Anmeldenummer: PCT/EP2006/000527
(87) Internationale Veröffentlichungsnummer: WO 2006/089607

(56) Entgegenhaltungen:
- DE-A1- 2 621 909
- DE-A1- 10 239 743
- GB-A- 1 598 378
- GB-A- 2 289 416
- US-A- 4 186 728

## Beschreibung

Die Erfindung betrifft eine Vorrichtung zur Messung von Hirnparametern nach dem Oberbegriff des Anspruchs 1.

Eine derartige Vorrichtung ist bekannt aus der DE 102 39 743 A1. Von zentraler Bedeutung ist bei derartigen Hirnparametersensoren der feste und dauerhaft stationäre Sitz der Sensoreinheit am Schädelknochen. Jede Relativbewegung des Himparametersensors zum Hirngewebe muss nach erfolgter Implantation verhindert werden, um eine Himschädigung zu vermeiden. Die Befestigung der Sensoreinheit am Schädelknochen ist bei den bekannten Himparametersensoren entweder durch eine recht aufwändige Befestigungstechnik gelöst oder ist hinsichtlich der Sicherung gegen eine Relativbewegung nicht zufriedenstellend.

Aus der US 4,186,728 ist eine Hirndruck-Messvorrichtung mit einer Sensoreinheit bekannt, die über ein selbstschneidendes Außengewinde gegen einen Schädelknochen abdichtet. Die DE 26 21 909 A1 beschreibt eine Vorrichtung zur Messung des intrakraniellen Druckes im Gehirn. Die GB 1 598 378 beschreibt einen Gewindekörper zum Einschrauben in einen Schädelknochen. Die GB 2 289 416 A beschreibt eine Knochenschraube.

Es ist eine Aufgabe der vorliegenden Erfindung, eine Vorrichtung der eingangs genannten Art derart weiterzubilden, dass eine gegen eine Relativbewegung zum Schädelknochen gesicherte, dauerhafte und gleichzeitig unaufwendige Befestigung gewährleistet ist.

Diese Aufgabe ist erfindungsgemäß gelöst durch eine Vorrichtung mit den im Kennzeichnungsteil des Anspruchs 1 angegebenen Merkmalen.

Erfindungsgemäß wurde erkannt, dass eine unaufwändige Befestigung der Sensoreinheit am Schädelknochen dann erreicht werden kann, wenn die Durchführung der Sensoreinheit durch den Schädelknochen gleichzeitig als Befestigungsstelle genutzt wird. Hierzu dient das selbstschneidende Außengewinde, welches zu einem dauerhaften, sicheren und unaufwändigen Halt der Sensoreinheit am Schädelknochen führt. Eine unerwünschte Relativbewegung der Sensoreinheit zum Hirngewebe nach erfolgter Implantierung ist dann sicher verhindert. Aufwändige zusätzliche Befestigungsmaßnahmen können entfallen. Das Außengewinde, das ein integraler Bestandteil der Sensoreinheit ist, führt zu einer kompakten Vorrichtung. Gleichzeitig ist eine unerwünschte Verlagerung des Außengewindes gegenüber der Sensoreinheit sicher verhindert.

Ein Außengewinde nach Anspruch 2 löst ein weiteres zentrales Problem bei der Anordnung einer mittels Durchführung durch den Schädelknochen implantierbaren Messvorrichtung, nämlich die sichere Abdichtung der Sensoreinheit gegen den Schädelknochen, sodass ein Austreten von Flüssigkeit oder Hirngewebe sicher verhindert ist. Diese Abdichtung erfolgt bei der Anordnung mit einem dichtenden Außengewinde direkt im Bereich der Durchführung, sodass die Anforderungen an eine nachgelagerte Dichtung im proximalen Sensorabschnitt verringert sind beziehungsweise eine derartige nachgelagerte proximale Abdichtung gänzlich entfallen kann.

Ein Außengewinde nach Anspruch 3 ist in der Herstellung unaufwändig.

Ein Außengewinde nach Anspruch 4 ermöglicht eine funktionale Trennung der verschiedenen Funktionen dieses Außengewindes, nämlich Einschneiden einerseits und Abdichten andererseits.

Ein Anschlag nach Anspruch 5 erlaubt ein definiertes Einschrauben der Vorrichtung. Im Bereich des Anschlags ist zudem eine zusätzliche, dem Außengewinde nachgelagerte Abdichtung der Sensoreinheit am Schädelknochen im proximalen Sensorabschnitt möglich.

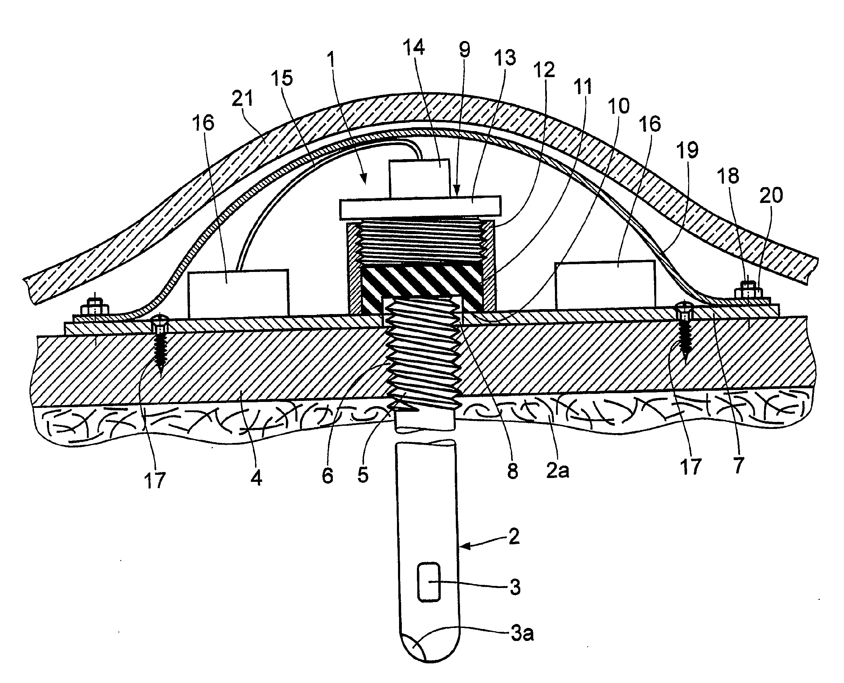

Ein Ausführungsbeispiel der Erfindung wird nachfolgend anhand der Zeichnung näher erläutert. Die einzige Figur zeigt einen Schnitt durch einen Kopfausschnitt eines Patienten mit einer Vorrichtung zur Messung von Hirnparametern.

Die insgesamt mit 1 bezeichnete Vorrichtung zur Messung von Hirnparametern umfasst eine Katheter-Sensoreinheit 2, die distal minimalinvasiv im Parenchym und/oder den Ventrikeln implantiert ist. An ihrem distalen Ende, also dort, wo die Katheter-Sensoreinheit 2 in Hirngewebe beziehungsweise Liquor 2a des Patienten hineinragt, weist die Katheter-Sensoreinheit 2 einen Drucksensor 3 und einen Temperatursensor 3a auf. Proximal ist die Katheter-Sensoreinheit 2 an einem Schädelknochen 4 des Patienten festgelegt.

Zur Festlegung der Katheter-Sensoreinheit 2 am Schädelknochen 4 weist ein proximaler Umfangsabschnitt der Sensoreinheit 2 ein selbstschneidendes Außengewinde 5 auf. Letzteres erzeugt beim Einschneiden in den Schädelknochen 4 in diesem ein zum Außengewinde 5 komplementäres Innengewinde 6.

Das Außengewinde 5 ist ein integraler Bestandteil der Sensoreinheit 2, ist also kein von diesem separates Bauteil. Das Außengewinde 5 weist eine in der Zeichnung nicht dargestellte dichtende Beschichtung auf, sodass über das Außengewinde 5 die Sensoreinheit 2 gegen den Schädelknochen 4 abgedichtet ist. Liquor oder Gewebe kann daher aus dem Hirn am Ort der implantierten Sensoreinheit 2 nicht austreten.

Zur Vorrichtung 1 gehört ferner eine Grundplatte 7 mit einer Durchgangsbohrung 8, durch welche die Katheter-Sensoreinheit 2 hindurchgeführt ist und die mit dem Innengewinde 6 fluchtet. An der Grundplatte 7 liegt bei vollständig eingeschraubter Sensoreinheit 2 ein proximales Kopfteil 9 über eine Erweiterungsstufe 10 an. Das Kopfteil 9 der Sensoreinheit 2 hat also einen deutlich größeren Umfang als der Katheterabschnitt der Sensoreinheit 2. Die Erweiterungsstufe 10 stellt einen an einem proximalen Endabschnitt, nämlich dem Kopfteil 9, ausgeführten Anschlag der Sensoreinheit 2 dar, der die Einschraubtiefe von letzterer begrenzt.

Im Kopfteil 9 ist eine zusätzliche proximale Dichtung 11 angeordnet, die in eine Hülse 12 des Kopfteils 9 eingesetzt ist. Auch die Dichtung 11 dichtet die Sensoreinheit 2 gegen den Schädelknochen 4 über die Grundplatte 7 ab. Aufgrund der Dichtungswirkung des Außengewebes 5 kann auf die Dichtung 11 auch verzichtet werden. Auf die Dichtung 11 kann mit Hilfe eines Schraubdeckels 13, der in der Zeichnung von oben her in die Hülse 12 eingeschraubt ist, Druck ausgeübt werden.

Über einen Mikrostecker 14 und eine Signalleitung 15 ist die Sensoreinheit 2 mit einer in der Zeichnung mehrteilig dargestellten Elektronikeinheit 16 verbunden.

Die Grundplatte 7 ist über Schrauben 17 am Schädelknochen 4 fixiert. Seitlich weist die Grundplatte 7 in der Zeichnung nach oben überstehende Gewindebolzen 18 auf. Über diese ist eine die Vorrichtung 1 in der Zeichnung nach oben abdeckende semiflexible Abdeckung 19 randseitig gesteckt. Die Abdeckung 19 ist dabei randseitig zwischen die Grundplatte 7 und Befestigungsmuttern 20, die auf die Gewindebolzen 18 geschraubt sind, geklemmt. Die Abdeckung 19 schützt insgesamt die Vorrichtung 1. Die Abdeckung 19 wird von Kopfhaut 21 überspannt.

Beim Implantieren der Sensoreinheit 2 wird zunächst eine Bohrung im Schädelknochen 4 ausgeführt. Anschließend wird die Grundplatte 7 so mit Hilfe der Schrauben 17 fixiert, dass die Durchgangsbohrung 8 mit der Bohrung im Schädelknochen 4 fluchtet. Sodann wird das Katheterteil der Sensoreinheit 2 durch die Bohrung im Schädelknochen 4 hindurchgeführt. Sobald das Außengewinde 5 beim Einführen die Bohrung im Schädelknochen 4 erreicht, wird das Außengewinde 5 eingeschraubt. Durch die selbstschneidende Wirkung des Außengewindes 5 entsteht aus der Bohrung im Schädelknochen 4 das Innengewinde 6. Der Einschraubvorgang wird fortgesetzt, bis die Hülse 12 an der Grundplatte 7 anliegt. Schließlich erfolgt, nach Aufschrauben des Schraubdeckels 13, noch die Verkabelung mit den auf der Grundplatte 7 bereitgestellten Modulen der Elektronikeinheit 16. Anschließend wird die Abdeckung 19 angebracht und die zunächst entfernte Kopfhaut 21 wieder über die Vorrichtung 1 gespannt. Diese ist dann zur Messung der entsprechenden physiologischen Parameter Hirndruck und Hirntemperatur über den Drucksensor 3 und den Temperatursensor 3a einsatzbereit.

## Patentansprüche

1. Vorrichtung (1) zur Messung von Hirnparametern
- mit einer Katheter-Sensoreinheit (2), die
- - distal minimalinvasiv im Parenchym und/oder den Ventrikeln unter Durchführung durch den Schädelknochen (4) implantierbar ist,
- - proximal am Schädelknochen (4) festlegbar ist,
- wobei
- - ein proximaler Umfangsabschnitt der Sensoreinheit (2) ein selbstschneidendes Außengewinde (5) zur Befestigung der Sensoreinheit (2) am Schädelknochen (4) aufweist,
- - das Außengewinde (5) ein integraler Bestandteil der Sensoreinheit (2) ist.

2. Vorrichtung nach Anspruch 1, **dadurch gekennzeichnet, dass** das Außengewinde (5) derart ausgeführt ist, dass über dieses die Sensoreinheit (2) gegen den Schädelknochen (4) abdichtbar ist.

3. Vorrichtung nach Anspruch 2, **dadurch gekennzeichnet, dass** das Außengewinde (5) aus einem dichtenden Material ausgeführt ist.

4. Vorrichtung nach Anspruch 2, **dadurch gekennzeichnet, dass** das Außengewinde (5) eine dichtende Beschichtung aufweist.

5. Vorrichtung nach einem der Ansprüche 1 bis 4, **dadurch gekennzeichnet, dass** die Sensoreinheit (2) an einem proximalen Endabschnitt (9) einen am Schädelknochen (4) anlegbaren Anschlag (10) aufweist.

## Claims

1. Device (1) for measuring brain parameters
- comprising a catheter sensor unit (2), which
- - can be implanted distally in a minimally-invasive manner in the parenchyma and/or the ventricles, guided through the cranial bone (4),
- - can be fixed proximally to the cranial bone (4),
- wherein
- - a proximal circumferential portion of the sensor unit (2) has a self-cutting outer screw thread (5) for fixing the sensor unit (2) to the cranial bone (4),
- - the outer screw thread (5) is an integral component of the sensor unit (2).

2. Device according to claim 1, **characterised in that** the outer screw thread (5) is configured such that the sensor unit (2) can be sealed from the cranial bone (4) by means of said outer screw thread.

3. Device according to claim 2, **characterised in that** the outer screw thread (5) is made of a sealing material.

4. Device according to claim 2, **characterised in that** the outer screw thread (5) has a sealing coating.

5. Device according to any one of claims 1 to 4, **characterised in that**, at a proximal end portion (9), the sensor unit (2) has a stop (10) which can be positioned against the cranial bone (4).

## Revendications

1. Dispositif (1) de mesure de paramètres cérébraux
- comprenant une unité de capteur-cathéter (2) qui
- - peut être implantée par voie mini-invasive dans le parenchyme et/ou dans les ventricules moyennant la réalisation d'un passage à travers l'os crânien (4),
- - peut être fixée de manière proximale à l'os crânien (4),
- où
- - une portion périphérique proximale de l'unité de capteur (2) présente un filetage externe (5) auto taraudant pour la fixation sur l'os crânien (4),
- - le filetage externe (5) constitue une partie intégrante de l'unité de capteur (2).

2. Dispositif selon la revendication 1 **caractérisé en ce que** le filetage externe (5) est conçu de telle manière que l'unité de capteur (2) peut être rendue étanche vis-à-vis de l'os crânien (4) par l'intermédiaire de celui-ci.

3. Dispositif selon la revendication 2 **caractérisé en ce que** le filetage externe (5) est conçu dans un matériau d'étanchéité.

4. Procédé selon la revendication 2 **caractérisé en ce que** le filetage externe (5) présente un revêtement d'étanchéité.

5. Dispositif selon l'une des revendications de 1 à 4 **caractérisé en ce que** l'unité de capteur (2) présente un épaulement (10) pouvant s'appuyer sur l'os crânien (4) au niveau d'une portion d'extrémité proximale (9).
